# EUROPEAN PATENT SPECIFICATION

(11) **EP 3 049 158 B1**
(45) Date of publication and mention of the grant of the patent: **23.10.2019**
(21) Application number: 14783743.9
(22) Date of filing: 26.09.2014
(51) Int. Cl.: A61Q 11/00, A61K 8/02, A61K 8/34, A61K 8/73

(54) **ORAL CARE COMPOSITION**
MUNDPFLEGEMITTEL
COMPOSITION POUR LA CAVITÉ ORALE

(30) Priority: 27.09.2013 US 201361883810 P
(43) Date of publication of application: 03.08.2016
(73) Proprietor: Rohm and Haas Company, Philadelphia, PA 19106 (US)
(72) Inventor: JOSHI, Kinjalbahen, Philadelphia, PA 19106-2399 (US); KEENAN, Andrea, Plymouth Meeting, PA 19462 (US)
(74) Representative: Houghton, Mark Phillip
(86) International application number: PCT/US2014/057677
(87) International publication number: WO 2015/048417

(56) References cited:
- EP-A1- 1 417 895
- EP-A2- 2 046 264
- EP-A2- 2 431 028
- WO-A1-2004/060335
- WO-A1-2014/000803
- WO-A2-2009/045022
- WO-A2-2010/138544
- WO-A2-2011/094497

## Description

### Field of the Invention

The invention relates to compositions for delivery of a payload under aqueous conditions.

### Description of Related Art

Compositions such as toothpastes used to deliver payloads under aqueous conditions have typically involved the use of one or more polymers in a single composition. In some cases, the composition requires that one of the polymers is water soluble and one is insoluble. Also, many previously disclosed polymer compositions dissolve under slightly aqueous conditions and would therefore not be stable in formulations such as toothpaste. Thus there is a need for polymer compositions that can incorporate a payload, be stable under aqueous conditions, and dissolve under highly aqueous conditions, to deliver a payload. Reference is made to international patent application WO 2009/045022 A2, European patent application EP 1417895 A1, WO 2014/000803 A1, EP 2431028 A2, WO 2010/138544 A2, WO 2011/094497 A2 and EP 2046264 A2.

### SUMMARY OF THE INVENTION

The disclosed invention relates to a composition for delivery of a payload to an oral cavity. The composition incorporates a payload and is substantially stable under slightly aqueous conditions, but dissolves when exposed to moderate or highly aqueous conditions, and/or mechanical action, releasing the payload.

The invention provides a toothpaste formulation comprising:
a toothpaste base having less than about 35% by weight water; and
a composition in the form of a film comprising:
   less than or equal to 5% water by weight of the film;
   a cellulose polymer having hydroxypropyl methylcellulose subunits and a viscosity at 20°C of 44 mPa·s to 60 mPa.s as a 2% solution in water, starch, a humectant and a payload, wherein
   the weight ratio of the cellulose polymer to the starch is about 3:1 to about 0.75:1.

Described herein are methods for making a film composition having:
a cellulose polymer as defined above, starch, a humectant and a payload; wherein
the weight ratio of cellulose polymer to starch is about 3:1 to about 0.75:1.

Further herein are methods for making a toothpaste formulation having:
a toothpaste base and a cellulose polymer composition, wherein
the toothpaste base has less than about 35% water by weight, and the composition includes:
   a cellulose polymer as defined above, starch, a humectant and a payload; wherein
   the weight ratio of cellulose polymer to starch is about 3:1 to about 0.75:1.

Also described herein is a method for releasing a payload from:
a composition having:
   a cellulose polymer as defined above, starch, a humectant and a payload; wherein
   the weight ratio of cellulose polymer to starch is about 3:1 to about 0.75:1, or from a toothpaste formulation having:
      a toothpaste base and a cellulose polymer composition, wherein
the toothpaste base has less than about 35% water by weight, and the composition includes:
   a cellulose polymer, as defined above, starch, a humectant and a payload; wherein
   the weight ratio of cellulose polymer to starch is about 3:1 to about 0.75:1.

These and other features and advantages of the present invention will be more fully understood from the following detailed description of the invention taken together with the claims. It is noted that the scope of the claims is defined by the recitations therein and not by the specific discussion of features and advantages set forth in the present description.

### DETAILED DESCRIPTION OF THE INVENTION

The term "polymer" as used herein, is synonymous with "copolymer", "heteropolymer" and "alternating copolymer" and means a large molecule (macromolecule) composed of a repeating series of one or more alternating monomeric species. These sub-units are typically connected by covalent chemical bonds.

The term "alkyl" as used herein, means a straight or branched chain hydrocarbon containing from 1 to 10 carbon atoms unless otherwise specified. The term alkyl includes both saturated and unsaturated hydrocarbons of from 1 to 10 carbon atoms. Saturated alkyl refers to hydrocarbon groups of, for example, 1-10 carbon atoms, and having no sites of unsaturation. Unsaturated alkyl refers to hydrocarbon groups of, for example, 1-10 carbon atoms, and having one or more sites of unsaturation, and includes both alkenyl and alkynyl groups. Alkyl groups may be optionally substituted as described herein.

The term "alkoxy" as used herein, means an alkyl group, as defined herein, appended to the parent molecular moiety through an oxygen atom.

The term "substantially stable" means that the composition does not substantially fold, curl, dissolve or release a substantial amount of the payload under the prescribed environmental conditions. A small about of payload leaching may be observed under some conditions, but the composition is "substantially stable" if the composition retains greater than about 90 % of the payload over an allotted period of time, or the composition remains substantially unchanged upon visual inspection.

The term "slightly aqueous conditions" means conditions or an environment where the moisture content is less than about 35 % water by weight. Examples include a sealed container having a typical commercial toothpaste formulation.

The term "moderately aqueous conditions" means conditions or an environment where the moisture content is about 35 % to about 50 % water by weight.

The term "highly aqueous conditions" means conditions or an environment where the moisture content is greater than about 50 % water by weight. Examples include an oral cavity or the conditions that occur when a toothpaste formulation is used to brush teeth.

The invention provides a composition having:
a cellulose polymer, starch, a humectant and a payload; wherein
the weight ratio of cellulose polymer to starch is about 3:1 to about 0.75:1.

The cellulose polymer includes hydroxypropyl methylcellulose subunits.

The cellulose polymer has a viscosity between about 44 and about 60 mPa·s as a 2 %solution in water at 20° C.

In some embodiments, the cellulose polymer is hot water dispersible and/or has a viscosity greater than about 45 mPa·s as a 2 % solution in water.

In certain embodiments, the hydroxypropyl methylcellulose polymer has a viscosity of about 45 mPa·s to about 60 mPa·s as a 2 % solution in water. In some embodiments, the hydroxypropyl methyl cellulose polymer has a viscosity of about 50 mPa·s as a 2 % solution in water at 20° C.

In some embodiments, the hydroxypropyl methyl cellulose polymer is of the formula: 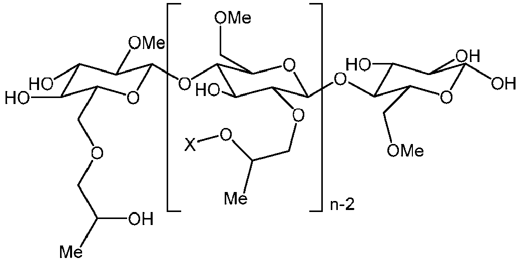 where X is independently hydrogen or alkyl. In certain embodiments X is C₁-C₆alkyl, such as methyl, ethyl, propyl, butyl, pentyl, isopropyl or isobutyl. In some embodiments, X is alkyl substituted with one to four alkoxy groups.

In some embodiments, the hydroxypropyl methyl cellulose polymer contains about 15 % to about 35 % methoxy groups and about 5 % to about 30 % of hydroxy groups. In other embodiments, the hydroxypropyl methyl cellulose polymer contains about 29 % methoxy groups and about 8.5 % of hydroxy groups.

In certain embodiments, the percentage of methoxy and hydroxy groups are (percentages are provided as approximate values):

| HPMC Polymer | % Methoxy | % Hydroxy |
|---|---|---|
| 1 | 29 | 8.5 |
| 2 | 28 | 5.0 |
| 3 | 18 | 27 |
| 4 | 22 | 8.1 |
| 5 | 25 | 25 |

In some embodiments, the starch is a carbohydrate consisting of a large number of glucose units joined by glycosidic bonds. The starch can be a white, tasteless and odorless powder that is insoluble in cold water or alcohol. Starches consist of two types of molecules: a linear and helical amylose and a branched amylopectin. The ratio of the two molecules varies depending on the plant species producing the starch, but is generally about 20 % to about 25 % amylose and about 75 % to about 80 % amylopectin by weight. But high amylopectin (up to about 100% amylopectin) and high amylose (up to about 50 % amylose) varieties are available and can be used in some embodiments.

In some embodiments, the starch is obtained from com, wheat, rice, tapioca, or potatoes. In a specific embodiment, the starch is corn starch or corn flour. In some embodiments, the corn starch is about 25 % amylose. In other embodiments, the corn starch is about 50 % amylose or 100% amylopectin. In other embodiments, the starch is a modified starch or a combination of starches. Numerous modified starches are known in the art, including, but not limited to, dextrin, alkaline-modified starch, bleached starch, oxidized starch, enzyme-treated starch, acetylated starch, hydroxypropylated starch, and mixtures thereof.

In some embodiments, the cellulose polymer is about 10 % to about 60 % of the composition by weight. In some embodiments, the polymer is 15 % to about 40 % by weight.

In some embodiments, the starch is about 10 % to about 60 % of the composition by weight. In other embodiments, the starch is about 15 % to about 40 % by weight.

In another embodiment, the weight ratio of cellulose polymer to starch in the composition is about 3:1 to about 0.75:1. In some embodiments, the weight ratio is about 2:1.

In another embodiment, the composition has a water content equal to or less than about 5 percent by weight.

The humectant can be chosen from diols, diol analogs, triols, triol analogs, polymeric polyols, or mixtures thereof. Numerous humectants are known in the art. A non-limiting list of example humectants includes glycols, such as propylene glycol, hexylene glycol and butylene glycol, glyceryl triacetate, vinyl alcohol, neoagarobiose, sugar polyols such as glycerol, sorbitol, xylitol and maltitol, polymeric polyols such as, polydextrose, quillaia, urea, glycerin, aloe vera gel, 2-methyl-1,3-propandiol (mp diol), alpha hydroxy acids such as lactic acid, and honey. In certain examples, the humectant can be glycerin or polysorbate

The humectant can also be a surfactant. The surfactant can be nonionic, anion, cationic or zwitterionic. A non-limiting list of example surfactants includes monoglycerides, lecithins, glycolipids, fatty alcohols, fatty acids, polysaccharides, sorbitan esters and polysorbates (polysorbate 20, 40, 60, 65 and 80, for example). In certain embodiments, the humectant can be a polysorbate, such as polysorbate 20 or polysorbate 80, or a mixture including one or more polysorbates.

In some embodiments, the humectant can include a combination of one or more humectants. For example, in some instances the humectant can be a mixture of a polyol and a surfactant, such as polysorbate 20 and glycerin.

The humectant can be present in the composition in an amount ranging from about 2 to about 15% by weight. The amount can be varied to obtain the desired properties of the resulting composition, such as stability in slightly aqueous environments, dissolution in highly aqueous environments, and the ability to incorporate a payload and optional additives. In some embodiments, the humectant is present as about 2 % to about 5 %, or about 5 % to about 8 %, or about 8 % to about 12 %, or about 12 % to about 15 % of the composition by weight. In certain embodiments, the humectant can be about 5 % to about 10 % of the composition by weight.

In another embodiment, the further comprises one or more additives. The additive can be any component added to obtain a desired property of the resulting composition. Although the additive may be released upon dissolution of the composition in a highly aqueous environment, unlike the payload, a well-chosen additive does not have any significant interaction with or use in the environment. Additives can include, coloring agents and preservatives.

Coloring agents are used in amounts effective to produce the desired color and include natural food colors and dyes suitable for food, drug and cosmetic applications (FD&C dyes). The coloring agents may be water-soluble, and include, in a non-limiting listing, Blue No. 1 (ethyl-[4-[[4-[ethyl-[(3-sulfophenyl)methyl]amino]phenyl]-(2-sulfophenyl)methylidene]-1-cyclohexa-2,5-dienylidene]-[(3-sulfophenyl)methyl]azanium), FD&C Blue No. 2 (disodium salt of 5,5-indigotindisulfonic acid), Green No. 3 (ethyl-[4-[[4-[ethyl-[(3-sulfophenyl)methyl]amino]phenyl]-(4-hydroxy-2-sulfophenyl)methylidene]-1-cyclohexa-2,5-dienylidene]-[(3-sulfophenyl)methyl]azanium), Red No. 40 (disodium 6-hydroxy-5-((2-methoxy-5-methyl-4-sulfophenyl)azo)-2-naphthalenesulfonate) Red No. 3 (2-(6-Hydroxy-2,4,5,7-tetraiodo-3-oxo-xanthen-9-yl)benzoic acid) Yellow No. 5 (trisodium 1-(4-sulfonatophenyl)-4-(4-sulfonatophenylazo)-5-pyrazolone-3-carboxylate)) and Yellow No. 6 (Disodium 6-hydroxy-5-[(4-sulfophenyl)azo]-2-naphthalenesulfonate). In some embodiments, the coloring agent is titanium dioxide (TiO₂). The coloring agent may include a mixture of coloring agents. The amount of coloring agent used in the composition is determined depending on the color desired and the extent of the color desired.

In some embodiments, the additive can be a preservative. The choice of preservative will depend on the desired properties of the preservative. Various preservatives are known in the art, non-limiting examples include sodium benzoate and potassium sorbate. A preservative, or combination thereof, can be added in amounts of about 0.001 wt % to about 5 wt %, preferably of about 0.01 wt % to about 1 wt % of the film.

The payload can be any component added to the composition that is desired to be released upon dissolution of the composition in a highly aqueous environment. The payload can interact with or play a role in the environment.

In some embodiments, the payload is one or more of a sweetening agent, flavoring agent, breath-freshening agent, anti-microbial agent, herbal agent, botanical agent, vitamin, anti-oxidant, or mixture thereof.

Flavoring agents that can be used include those known in the art, such as natural and artificial flavors. Flavoring oils may be chosen from natural and synthetic flavoring oils, aromatics, oleo resins, and extracts derived from plants, leaves, flowers, fruits etc., and combinations thereof. Flavoring oils can include: spearmint oil, cinnamon oil, peppermint oil, clove oil, bay oil, thyme oil, cedar leaf oil, oil of nutmeg, oil of sage, and oil of bitter almonds. Flavoring agents can be used individually or in admixture. Commonly used flavors include mints such as peppermint, artificial vanilla, cinnamon derivatives, coffee, cocoa, and various fruit flavors, whether employed individually or in admixture. Any flavoring or food additive approved for use in food processing may be used. The amount of flavoring agent used in the composition is determined depending on the type and desired strength of the flavor.

Sweetening agents include both natural and artificial sweeteners. Suitable sweetener include water soluble sweetening agents such as monosaccharides, disaccharides and polysaccharides such as xylose, ribose, glucose (dextrose), mannose, galactose, fructose (levulose), sucrose (sugar), maltose, water soluble artificial sweeteners such as the soluble saccharin salts, i.e., sodium or calcium saccharin salts, cyclamate salts dipeptide based sweeteners, such a L-aspartic acid derived sweeteners, such as L-aspartyl-L-phenylalanine methyl ester (aspartame). The amount of sweetening agent used in the composition is determined depending on the type and desired sweetness. Breath-freshening agents can include zinc gluconate, zinc citrate and/or alpha ionone. These agents function in masking mouth odor and reducing volatile odor causing bacterial sulfur compounds. Also, several of the sweetening, flavoring and herbal agents described herein can be used as breath-freshening agents.

Anti-microbial agents include compounds that upon release from the composition interact with microbes in the environment, such as an antibacterial or antifungal agent. Examples include antibiotics, such as tricloscan, and antifungals, such as polyenes and azoles (imidazoles, triazoles and thiazoles).

Herbal and botanical agents and include plant roots, stems, roots, tuber, extracts, etc. that have a use, or perceived use, as a health supplement. Breath-freshening herbs include sage, myrrh, clove, coriander, ginger, cumin, fennel, peelu, tea tree oil or peppermint oil. Other herbs and botanicals have use for a variety of physiological effects, ranging from alertness to anti-hypertension.

The physiologically active components in the payload can be absorbed through the blood vessels in the oral activity and enter the blood stream. In some embodiments, the payload includes vitamins, minerals, amino acids, anti-oxidants, or a combination thereof. Vitamins, minerals, amino acids, anti-oxidants as used in the composition are organic compounds (or metal salts in the case of minerals) required by an organism as a vital or beneficial nutrient. In most cases, the organic compound or metal salt cannot be synthesized in sufficient quantities by the organism and must be obtained from the diet. Supplementation of vitamins, amino acids and anti-oxidants can be useful in the case of a deficiency. In some embodiments, the payload is an organic compound that increases alertness, such as caffeine, a mixture of B vitamins or combination thereof.

The amount of the payload incorporated into the composition is determined depending on the type of payload and the desired action of the payload following dissolution of the composition. The payload can be incorporated in the composition at a concentration of about 0.1 % to about 5.0 % by weight. In some embodiments, the concentration of the payload is 0.1 % to about 2.5 % or about 3.0 % to about 5.0 %. In some embodiments, the concentration of the payload is about 1.0 to about 3.0 % of the composition by weight.

The film is 75 to 125 microns in thickness.

The film can be segmented into fragments or flakes, or into a desired geometric shape, such as squares, rectangles or strips by die-cutting or slitting-and-die-cutting. The width and length of the film can vary according to the intended application. The dimensions of the film can vary from about 0.1 to about 10 millimeter in width and about 0.1 to about 10 millimeters in length. The ratio of length to width can be varied depending on the desired shape.

Commercial toothpaste typically has a water content of about 25 % to about 35% by weight. The compositions of the invention are stable below about 35 % water content by weight, so toothpaste formulations can be made using toothpaste bases having a water content sufficient to produce a toothpaste formulation having less than about 35% water by weight. In some embodiments, the toothpaste base has about 28 % to about 32 % water content. In other embodiments the toothpaste base has about 30 % water by weight.

In another embodiment, the composition is about 10 % to about 30 % by weight of the toothpaste formulation. In some embodiments, the composition is about 10 % to about 15 %, about 15 % to about 20 %, about 15 % to about 20 %, about 20 % to about 25 %, or about 25 % to about 30 % of the formulation by weight.

In some embodiments, the toothpaste formulation further includes an additive as described herein.

The invention provides a method for making a film composition, the method comprising:
forming a mixture of cellulose polymer in water;
adding one or more humectants to the mixture;
adding starch to the mixture;
allowing the mixture to cool; and
removing water from the cooled mixture to provide a composition having less than about 5% water by weight.

The invention also provides a method for making a toothpaste formulation, the method comprising:
forming a mixture of cellulose polymer in water;
adding one or more humectants to the mixture;
adding starch to the mixture;
allowing the mixture to cool;
removing water from the cooled mixture to provide a solid composition; and
adding the solid composition to a toothpaste base.

In some embodiments of the method for making a film composition or a toothpaste formulation, the mixture can be formed in water or a buffered solution. In some instances, the mixture can be heated and/or stirred. The mixture can be heated, in some instances to about 90 to about 100 °C. The addition of starch as the last component to the mixture can provide better holding characteristics during the subsequent water removal step.

In some embodiments of the method for making a film composition or a toothpaste formulation, the method further comprises adding a payload to the mixture.

In some embodiments, the method further includes adding an additive to the mixture. In some embodiments, the additive can be added to the toothpaste base.

In another embodiment, the removal of water comprises casting the cooled mixture to provide a film. The casting can involve forming a layer of the cooled mixture and drying. The layer can be about 50 to about 250 microns thick, or about 50 to about 100 microns, or about 150 to about 250 microns, or about 100 to about 150 microns. The solid composition can be fragmented in a number of ways before being added to the toothpaste base.

The invention further provides a method for releasing a payload from an oral care composition, the method comprising:
subjecting a composition to mechanical action or a wet environment,
wherein
the composition comprises a cellulose polymer, starch, a humectant and a payload, wherein
the weight ratio of cellulose polymer to starch is about 3:1 to about 0.75:1; and
the wet environment comprises greater than about 35 % water by weight.

In some embodiments, the composition is a component in a toothpaste formulation described herein, which comprises less than about 35 % water by weight.

In some embodiments, the mechanical action comprises the brushing of teeth.

In some embodiments, the wet environment is the addition of water to the toothpaste formulation. In other embodiments, the wet environment is an oral cavity.

In some embodiments, the composition incorporates a payload that is delivered upon subjection to the mechanical action or wet environment. The payload can be as described herein.

### EXAMPLES

### Example 1: Film Composition

| | Weight Percentage of Mixture | Weight Percentage of Dried Composition |
|---|---|---|
| Water | 76.5 | 0 |
| Hydroxypropyl methylcellulose | 7.5 | 32 |
| Titanium Dioxide | 1 | 4.2 |
| Glycerin | 9.5 | 40.4 |
| Polysorbate 20 | 1.5 | 6.4 |
| Corn Starch | 4 | 17 |

Hydroxypropyl methylcellulose and titanium dioxide are added to hot water (90-95 °C) and stirred for 100-120 minutes while the temperature is allowed to gradually decrease. Glycerin and polysorbate 20 are added and stirring continued for 40-50 minutes. Corn starch is added, and the mixture stirred for 40-50 minutes. The resulting mixture is cast into a layer 100-115 microns thick and dried at 90 °C for 20 minutes. The moisture content of the resulting film is < 5 % by weight.

### Example 2: Toothpaste Formulation

The film formed in Example 1 is cut into squares (1.5mm×1.5mm), combined with a toothpaste base that has less than 33 % water content by weight, and the resulting toothpaste mixed in an overhead mixer at 100-120 rpm.

### Example 3: Stability of Composition in Toothpaste Formulation

A toothpaste formulation was formed by combining the film composition from Example 1 with a toothpaste base that has less than 33 % water content by weight, split and A) stirred at room temperature and B) stirred at 40 °C. After one month, the film compositions are stable in the toothpaste formulations stirred at both A) room temperature and B) 40 °C. No curling or swelling was observed. Upon subjection to mechanical action with brushing, the film compositions readily dissolved.

## Claims

1. A toothpaste formulation comprising:
a toothpaste base having less than 35% by weight water; and
a composition in the form of a film comprising:
less than or equal to 5% water by weight of the film;
a cellulose polymer having hydroxypropyl methylcellulose subunits and a viscosity at 20°C of 44mPa·s to 60 mPa·s as a 2% solution in water;
starch;
a humectant; and
a payload,
wherein the weight ratio of the cellulose polymer to the starch is 3:1 to 0.75:1, and
the film is 75 to 125 microns in thickness.

2. The toothpaste formulation of claim 1, where the weight ratio of the cellulose polymer to the starch is about 2:1.

3. The toothpaste formulation of claim 1 or claim 2, where the humectant is chosen from diols, diol analogs, triols, triol analogs, polymeric polyols, and mixtures thereof.

4. The toothpaste formulation of any of claims 1-3, where the composition further comprises an additive.

5. The toothpaste formulation of claim 4, where the additive is a coloring agent.

6. The toothpaste formulation of any of claims 1-5, where the payload is one or more of a sweeting agent, flavoring agent, coloring agent, herbal agent, botanical agent, vitamin and anti-oxidant.

7. The toothpaste formulation of any of claims 1-5, where the payload is caffeine.

8. The toothpaste formulation of any of claims 1-5, where the payload is 0.1 to 5.0 % of the composition by weight.

9. The toothpaste formulation of any of claims 1-5, wherein the composition is 10 % to 30 % of the toothpaste formulation by weight.

## Patentansprüche

1. Eine Zahnpastaformulierung, die Folgendes beinhaltet:
eine Zahnpastabasis mit weniger als 35 Gew.-% Wasser; und
eine Zusammensetzung in Form einer Folie, die Folgendes beinhaltet:
zu weniger als oder gleich 5 Gew.-% der Folie Wasser;
ein Cellulosepolymer mit Hydroxypropylmethylcellulose-Untereinheiten und einer Viskosität bei 20 °C von 44 mPa·s bis 60 mPa·s als 2%ige Lösung in Wasser;
Stärke;
ein Feuchthaltemittel; und
eine Nutzlast,
wobei das Gewichtsverhältnis des Cellulosepolymers zu der Stärke 3 : 1 bis 0,75 : 1 beträgt, und
die Folie eine Dicke von 75 bis 125 Mikrometer aufweist.

2. Zahnpastaformulierung gemäß Anspruch 1, wobei das Gewichtsverhältnis des Cellulosepolymers zu der Stärke etwa 2 : 1 beträgt.

3. Zahnpastaformulierung gemäß Anspruch 1 oder Anspruch 2, wobei das Feuchthaltemittel ausgewählt ist aus Diolen, Diolanaloga, Triolen, Triolanaloga, polymeren Polyolen und Mischungen davon.

4. Zahnpastaformulierung gemäß einem der Ansprüche 1-3, wobei die Zusammensetzung ferner ein Additiv beinhaltet.

5. Zahnpastaformulierung gemäß Anspruch 4, wobei das Additiv ein Färbemittel ist.

6. Zahnpastaformulierung gemäß einem der Ansprüche 1-5, wobei die Nutzlast ein Süßungsmittel, ein Aromastoff, ein Farbmittel, ein Kräutermittel, ein Pflanzenmittel, ein Vitamin und Antioxidationsmittel ist.

7. Zahnpastaformulierung gemäß einem der Ansprüche 1-5, wobei die Nutzlast Koffein ist.

8. Zahnpastaformulierung gemäß einem der Ansprüche 1-5, wobei die Nutzlast 0,1 bis 5,0 Gew.-% der Zusammensetzung beträgt.

9. Zahnpastaformulierung gemäß einem der Ansprüche 1-5, wobei die Zusammensetzung 10 Gew.-% bis 30 Gew.-% der Zahnpastaformulierung beträgt.

## Revendications

1. Une formulation de dentifrice comprenant :
une base de dentifrice ayant moins de 35 % en poids d'eau ; et
une composition sous la forme d'un film comprenant :
moins de ou une quantité égale à 5 % d'eau en poids du film ;
un polymère de cellulose ayant des sous-unités d'hydroxypropyl méthylcellulose et une viscosité à 20 °C de 44 mPa·s à 60 mPa·s en tant que solution à 2 % dans l'eau ;
de l'amidon ;
un humectant ; et
une charge utile,
dans laquelle le rapport en poids du polymère de cellulose à l'amidon est de 3/1 à 0,75/1, et
le film fait 75 à 125 microns d'épaisseur.

2. La formulation de dentifrice de la revendication 1, où le rapport en poids du polymère de cellulose à l'amidon est d'environ 2/1.

3. La formulation de dentifrice de la revendication 1 ou de la revendication 2, où l'humectant est choisi parmi des diols, des analogues de diol, des triols, des analogues de triol, des polyols polymères, et des mélanges de ceux-ci.

4. La formulation de dentifrice de n'importe lesquelles des revendications 1 à 3, où la composition comprend en outre un additif.

5. La formulation de dentifrice de la revendication 4, où l'additif est un agent de coloration.

6. La formulation de dentifrice de n'importe lesquelles des revendications 1 à 5, où la charge utile est un ou plusieurs éléments parmi un agent sucrant, un agent aromatisant, un agent de coloration, un agent d'origine végétale, un agent botanique, une vitamine et un antioxydant.

7. La formulation de dentifrice de n'importe lesquelles des revendications 1 à 5, où la charge utile est la caféine.

8. La formulation de dentifrice de n'importe lesquelles des revendications 1 à 5, où la charge utile représente de 0,1 à 5,0 % de la composition en poids.

9. La formulation de dentifrice de n'importe lesquelles des revendications 1 à 5, dans laquelle la composition représente de 10 % à 30 % de la formulation de dentifrice en poids.
